# EUROPÄISCHE PATENTSCHRIFT

(11) **EP 2 100 869 B1**
(45) Veröffentlichungstag und Bekanntmachung des Hinweises auf die Patenterteilung: **27.11.2019**
(21) Anmeldenummer: 09003469.5
(22) Anmeldetag: 10.03.2009
(51) Int. Cl.: C07C 29/151, C07C 31/04, F01K 23/06

(54) **Verfahren zur Herstellung von Methanol durch Verwertung von Kohlendioxid aus Abgasen fossil betriebener Energieerzeugungsanlagen**
Method for producing methanol by recovering carbon dioxide from exhaust gases of energy generation facilities powered by fossil fuels
Procédé de fabrication de méthanol en utilisant du dioxyde de carbone à partir de gaz d'échappement d'installations de production d'énergie à fonctionnement fossile

(30) Priorität: 10.03.2008 DE 102008013370; 04.02.2009 DE 102009007567
(43) Veröffentlichungstag der Anmeldung: 16.09.2009
(73) Patentinhaber: Harzfeld, Edgar, 18435 Stralsund (DE)
(72) Erfinder: Harzfeld, Edgar, 18435 Stralsund (DE)
(74) Vertreter: Garrels, Sabine

(56) Entgegenhaltungen:
- EP-A- 0 413 199
- EP-A- 0 497 226
- EP-A- 0 539 244
- WO-A-00/25380
- WO-A-2005/056737
- WO-A1-2007/058608
- DE-A1- 4 332 789
- DE-A1- 4 332 790
- DE-A1- 19 802 660
- JP-A- 57 167 935
- US-A1- 2007 254 969

## Beschreibung

Die Erfindung betrifft ein Verfahren zur Herstellung von Methanol durch Verwertung von Kohlendioxid aus Abgasen, insbesondere fossil betriebener Energieerzeugungsanlagen.

Im Stand der Technik ist es bekannt, dass Rauchgaskohlendioxid aus fossil befeuerten Heizwerken, Heizkraftwerken, Kraftwerken, Zementanlagen, Anlagen der Chemie, der Hüttenindustrie und so weiter in die Atmosphäre emittiert wird und dadurch einen Klimaschaden erzeugt. Das emittierte Kohlendioxid stellt demnach einen Klimaschadstoff dar. Zur Reduzierung der Kohlendioxidemissionen werden Forschungen betrieben, um das Kohlendioxid aus dem Rauchgas von Heizwerken, Kraftwerken oder Heizkraftwerken zu absorbieren und im Untergrund, beispielsweise in Aquiferen, Salzstöcken, ausgebeuteten Erdöl- beziehungsweise Erdgaslagerstätten und so weiter zu speichern (Ankertechnologien). Das Verfahren zur Gewinnung und Speicherung des Kohlendioxids im Untergrund wird auch als Carbon-Capture-Storage (CCS-Verfahren) bezeichnet.

Bei neueren Kraftwerksprozessen ist auch ein Verfahren als Oxyfuel-Prozess bekannt. Diese neuen Kraftwerksprozesse zeichnen sich dadurch aus, dass durch den Einsatz von Sauerstoff an Stelle von Luft die Gewinnung von fast reinem Kohlendioxid (CO2) aus Rauchgas erreicht wird, dessen Verpressung im Untergrund enorm erleichtert ist. Ungünstig ist allerdings die Tatsache zu werten, dass derzeit kein geschlossenes Konzept zur Nutzung des gespeicherten Kohlendioxids existiert.

Im Stand der Technik ist es weiterhin bekannt, dass der Ausbau der Anlagen zur Gewinnung regenerativer Energien, und hier insbesondere der Windenergie, einen außerordentlichen Aufschwung erfahren hat und zu einem unumkehrbaren Faktor bei der Energieversorgung geworden ist. Mit dem sogenannten Repowering und dem Aufbau von sogenannten Off-Shore-Anlagen wird diese Entwicklung fortgesetzt und der Anteil der durch diese Anlagen erzeugten Energie an der gesamten erzeugten Energie weiter erhöht. Nachteilig dabei ist jedoch, dass die naturgegebene Angebotssituation (auch als Dargebotssituation bezeichnet) der Windenergieeinspeisung sehr schwierig zu prognostizieren ist. Dadurch treten aufgrund der witterungsbedingten Unwägbarkeiten entweder Energieerzeugungsspitzen oder aber auch Energieerzeugungstäler beispielsweise in Windflautezeiten auf. Dadurch ist es gegeben, dass die konventionell betriebenen Kraftwerke in Spitzenlast- bzw. Spitzenabnahmezeiten bei Windflaute mit Höchstleistung produzieren muss, während beispielsweise in Zeiten, in welchen ausreichend Wind zur Verfügung steht, die Energieabnahme nicht so günstig ist, diese konventionellen Kraftwerke heruntergefahren werden müssen. Der Nachteil dabei ist, dass mit Unterlast gefahrene konventionelle Kraftwerke einen höheren CO2-Ausstoß und auch einen höheren CO-Ausstoß verursachen, als dies üblicherweise der Fall ist.

Im Stand der Technik ist es auch bekannt, mit Hilfe einer Elektrolyse Wasser in Wasserstoff und Sauerstoff aufzuspalten. Das Problem ist allerdings, dass es äußerst umständlich bzw. schwierig ist, den dabei erzeugten Wasserstoff zu lagern und insbesondere an die Stellen zu transportieren, an denen er benötigt wird. Des Weiteren ist es nachteilig, dass der bei der Elektrolyse anfallende Sauerstoff nur bedingt einer Verwertung zugeführt wird. Im Stand der Technik ist es weiterhin bekannt, Methanol aus Kohlendioxid und Wasserstoff unter Verwendung von Katalysatoren in Methanol umzusetzen.

In EP 0539244 A1 wird ein weitgehend geschlossenes System beschrieben, in dem in einer Elektrolyse unter Zufuhr von Energie Wasser in Wasserstoff und Sauerstoff zerlegt wird, in einer Methanolsynthese Methanol aus Kohlendioxid und Wasserstoff erzeugt wird, das Methanol einer Verbrennung zugeführt wird, wobei Energie frei gesetzt wird und das dabei entstehende Kohlendioxid und Wasser dem System wieder zugeführt wird. Basis der regenerativen Energiegewinnung ist die Photovoltaik. Das geschlossene System beinhaltet eine Tag- und eine Nacht-Lösung. Während solare Energie am Tag für die Elektrolyse genutzt wird, um Elektrolyse-Wasserstoff zu gewinnen, und CO₂ aus der Verbrennung von Methanol gewonnen wird, die beide der Methanolsynthese zugeführt werden, um Methanol zu produzieren, wird während des Nachtbetriebes Überschussstrom der Elektrolyse und dem Methanol-Reformer zugeführt, wodurch Elektrolysewasserstoff und CO₂ produziert werden, die der Methanolsynthese zugeführt werden.

Andere Veröffentlichungen befassen sich mit der Nutzung von Abgas aus einem Verbrennungsprozess zur Herstellung von Methanol (JP S57167935 A, DE 4332790 A1, US 2007/254969 A1). Die beschriebene Erfindung in JP S57167935 A betrifft eine effiziente Nutzung von Erdgas, das zur Wärme- und Stromerzeugung sowie zur Herstellung von Methanol genutzt wird. DE 4332790 A1 betrifft ein Verfahren zur Herstellung von Methanol aus Kohlendioxid und Wasserstoff. Es wird die Durchströmung des Katalysators optimiert._US 2007/254969 A1 betrifft ein effizientes und selektives chemisches Recycling von Kohlendioxiden zu Methanol, Dimethylether und daraus erzeugte Produkte. Es werden Kohlendioxidquellen wie Kraftwerke und Zementwerke genannt. Die zur Wasserstoffproduktion erforderliche Energie soll aus fossilen Energieträgern, Solar- oder Windenergie oder Geothermie hervorgehen. Das bei der Methanolverwertung (Verbrennung) entstehende Kohlendioxid soll einem Kreislauf zugeführt werden und wieder verwertet werden.

Die Lösung in EP 497226 A2 betrifft eine Methode zur Herstellung von Methanol, bei der in einem Kernkraftwerk produzierter Dampf in einem Dampfelektrolyseur zu Wasserstoff gewandelt und mit CO₂ aus einer CO₂-Quelle einer Methanolsynthese zugeführt wird. Dieser Prozess soll auch die Möglichkeit beinhalten, CO₂ und H₂ in CO und Dampf wandeln zu können. Weiterhin wird mit Sauerstoff aus der Dampfelektrolyse eine Druckluft erzeugt, die einer Kraftwerkseinheit zugeführt wird.

EP 413199 A1 betrifft eine Methode zur Reduktion der CO₂-Emissionen eines GuD-Kraftwerkes. In einem der Brennkammer einer Gasturbine vor geschalteten Vergaser wird Rohgas, bestehend aus CO₂, Schwefelverbindungen und Wasserstoff, hergestellt. Das Rohgas wird aufbereitet, wobei alle Verbindungen, außer Wasserstoff, aus dem Gas entfernt werden. Das entstehende Reingas, Wasserstoff, wird dann der Brennkammer der Gasturbine zugeführt. Der Wasserstoff und Luftsauerstoff reagieren zu Wasserdampf. Das während der Gasreinigung gewonnene CO₂ wird entsorgt oder u.a. einer Methanolerzeugung zugeführt. Das Methanol wird für die Verbrennung in der Brennkammer der Gasturbine vorgesehen. Weiterhin soll Methanol dazu dienen, einen Spitzenbedarf in der Stromerzeugung zu decken.

DE 4332789 A1 bezieht sich auf ein Verfahren zur Speicherung von Energie. Sie betrifft insbesondere ein Verfahren zur Speicherung von Energie in Form von Wasserstoff.

Die Lösung in DE 19802660 A1 betrifft ein Verfahren zur Kombination eines GuD-Prozesses mit einem Herstellungsprozess zur Produktion von Methanol. Zusätzlich wird PV-Strom für eine Wasserstoffelektrolyse und Biogas/Abgas-CO2 für eine Methanolsynthese verwendet.

WO 2000/025380 A2 befasst sich mit einem System und ein Verfahren zur Speicherung von elektrischer Energie in Wasserstoff und Methanol.

WO 2005/056737 A1 betrifft eine Methode und eine Installation zur Erzeugung flüssiger Energieträger aus festen Kohlenstoff-Energieträgern. Für die Elektrolyseeinheit wird externe Energie aus erneuerbaren Energien bezogen. Es wird immer ein Maximum an erneuerbaren Energien bezogen. Externe Energie aus erneuerbaren Energien soll in Methanol gespeichert werden.

Der Grundgedanke in WO 2007/058608 A1 besteht in einem Verfahren und einem System, bei dem Energie z. B. aus Wind- und PV-Anlagen verwendet werden soll, um aus Kohlendioxid und Wasser Methylalkohol herzustellen. Für die Herstellung von Methylalkohol soll eine Brennstoffzelle bzw. eine elektrochemische Zelle verwendet werden. Weiterhin wird ein Verfahren zur Herstellung von Nanopartikeln beschrieben, die zur Beschichtung von Elektroden verwendet werden sollen und eine poröse Oberfläche erzeugen.

Ausgehend von dem zuvor beschriebenen Stand der Technik ist es Aufgabe der Erfindung, ein Verfahren und eine Energieerzeugungsanlage zur Herstellung von Methanol zur Verfügung zu stellen, das es ermöglicht, die Abgase fossil befeuerter Kraftwerke, Heizkraftwerke und anderer Emittenten in einer Methanolsynthese einzusetzen und unter Verwendung von regenerativ erzeugtem Wasserstoff und Katalysatoren in Methanol umzusetzen, wobei insbesondere eine flexible Anwendung realisiert werden soll. Ein Ziel ist es eine Möglichkeit der Speicherung der insbesondere durch Solar- und Windenergie zur Verfügung gestellten erneuerbaren Energien zur Verfügung zu stellen, wobei gleichzeitig die Umwelt von umweltschädigenden Kohlendioxid aus den Abgasen fossil betriebener Heizkraftwerke beziehungsweise Heizwerke entlastet werden soll.

Die Aufgabe der Erfindung wird gelöst durch ein Verfahren zur Herstellung von Methanol durch die Verwertung von Kohlendioxid aus Abgas fossil befeuerter Kraftwerke, Heizkraftwerke und Emittenten, ausgewählt aus Zementwerken der Zementindustrie, chemischen Anlagen, Stahlwerken, Anlagen der Industrie, Biogas- und/oder Biomasseanlagen, wobei Kohlendioxid aus dem Abgas für eine Methanolsynthese mit Wasserstoff vermischt und unter Verwendung von Katalysatoren in Methanol umgesetzt wird. Erfindungsgemäß ist eine technologische Einheit für eine Kohlendioxid-Verwertung und Wasserstoff-Methanolspeicherung (CV WMS) vorgesehen. Diese technologische Einheit der CO2-Verwertungs- und Wasserstoff-Methanol-Speicherung (CV WMS) besteht aus
- Elektrolyse von Wasser mit H2- und O2-Gewinnung,
- CO2 - Ad- oder Absorption mit CO2-Gewinnung und
- Methanolsynthese mit MeOH-Speicherung,
und wird containerisiert am Standort einer Kohlendioxid-Quelle zur Verfügung gestellt.

In einer Ausführung wird das Kohlendioxid aus dem Abgas der Methanolsynthese direkt zugeführt und/oder das Kohlendioxid wird aus Speichern der Methanolsynthese indirekt zugeführt. Dabei wird in einer weiteren Ausführung das Kohlendioxid aus dem Abgas der fossil befeuerten Kraftwerke absorbiert oder adsorbiert.

In einer weiteren Ausführung wird das hergestellte Methanol in den Feuerungsprozess von Kraftwerken zur Stromerzeugung zurückgeführt, um Windflauten auszugleichen. Das dabei frei werdende Kohlendioxid wird erneut zur Synthese von Methanol eingesetzt.

In einer weiteren Ausführung ist eine Steuerung vorgesehen, mittels derer die Erzeugung der Elektroenergie bedarfsgerecht gesteuert wird, wobei bei nicht ausreichenden Aufkommen aus Wind- , Wasser- und/oder Solarenergieerzeugungsanlagen die fossilen Energieerzeugungsanlagen mit Methanol aus dem Methanol-Speicher für die Erzeugung von Elektroenergie unterstützt werden.

Der Vorteil dieses Verfahrens ist der, dass neben der Entlastung der Umwelt durch das klimaschädliche Kohlendioxid gleichzeitig ein Energiespeicher in Form von Methanol zur Verfügung gestellt wird, der es ermöglicht, beispielsweise in Starklast- bzw. Hochlastphasen und bei nicht ausreichend zur Verfügung stehender Energie aus Windenergieerzeugungsanlagen oder Solarstromanlagen, dieses Methanol wieder in den Kraftwerksprozess einzusetzen. Die in Starkwindzeiten vorhandene Elektroenergie wird dabei allerdings gleichzeitig dazu verwendet, Wasserstoff durch eine Elektrolyse zu erzeugen und mittels des Kohlendioxids durch Vermischen und unter Verwendung von Katalysatoren in Methanol umzusetzen. Wasserstoff wird dabei durch elektrolytische Spaltung von Wasser mit Hilfe von Strom aus erneuerbaren Energiequellen, wie Wind, Solarstrahlung, Wasserkraft oder dergleichen, als regenerativer Wasserstoff gewonnen und ggf. gespeichert. Es wird damit ein geschlossener Prozess erhalten, der es ermöglicht, Schwachlast- beziehungsweise Starklast- bzw. Hochlastphasen derart abzupuffern, dass eine optimale Nutzung erneuerbarer Energien, wie der Windenergie gegeben ist und gleichzeitig CO2 -Emissionen zum Schutz der Umwelt reduziert werden.

Die Erfindung betrifft demnach den Verfahrensweg zur Verwertung von Kohlendioxid aus dem Abgas fossil befeuerter Heizkraftwerke und anderer Kohlendioxid-Emittenten mit Hilfe von Wasserstoff, der durch die Elektrolyse von Wasser mit Hilfe von elektrischem Strom aus regenerativen Energiequellen gewonnen wurde, zur Herstellung von Methanol. Es wird aber auch die Möglichkeit eröffnet, die "überschüssige" Elektroenergie in Form von Methanol zu speichern. Der bei der Aufspaltung von Wasser zu Wasserstoff und Sauerstoff sozusagen überschüssig zur Verfügung stehende Sauerstoff kann ebenfalls in dem Verbrennungsprozess der fossil befeuerten Kraftwerke wieder eingesetzt werden. Damit wird ein Ergebnis erreicht, wie es im Stand der Technik bisher noch nicht erreicht wurde, nämlich dass die Schadstoffe, nämlich das Kohlendioxid aus dem Abgas der Kraftwerke herausgezogen wird, gegebenenfalls gespeichert und dann in Zeiten von überschüssig vorhandener Elektroenergie mit Hilfe der Elektrolyse Wasserstoff erzeugt wird, der dann zu Methanol umgesetzt wird. Die Erfindung ermöglicht gleichzeitig den Einsatz des überschüssigen Sauerstoffs aus der Elektrolyse.

Um Starklast- und Schwachlastphasen weiter abzupuffern ist es selbstverständlich möglich, Kohlendioxid, Sauerstoff und Wasserstoff in entsprechenden Speicheranlagen vorzuhalten. Das mit der Methanolsynthese erzeugte Methanol kann ebenfalls in Speicheranlagen bevorratet gehalten werden, um es dann in entsprechenden Hochlastphasen, bei nicht ausreichend zur Verfügung stehender Elektroenergie aus erneuerbaren Energiequellen wieder dem Verbrennungsprozess zuzuführen. Das Methanol kann selbstverständlich auch ohne weiteres in entsprechende Behälter abgefüllt werden, um es an andere Orte zu transportieren. Hierzu ist nicht eine so komplizierte und insbesondere kein hohes Gefahrenpotential beinhaltende Transporttechnologie notwendig, wie beispielsweise bei dem Transport von Wasserstoff. Wasserstoff ist bekanntermaßen hochentzündlich und insbesondere hochexplosiv und es ist erforderlich, diesen Wasserstoff mit höchsten Sicherheitsbestimmungen nicht nur zu erzeugen und abzufüllen, sondern insbesondere auch zu transportieren. Dies ist bisher nur mit höheren Aufwendungen als bei Methanol möglich, so dass es derzeit kein tragbares Konzept gibt, diesen Wasserstoff entsprechend zu transportieren.

Das Verfahren zur Herstellung von Methanol beziehungsweise die Möglichkeit zur Speicherung von regenerativer Energie löst dieses Problem, indem es an einem einzigen Standort möglich ist, überschüssige Elektroenergie zu verwerten, indem Wasserstoff erzeugt wird und zum anderen gleichzeitig vorhandene Kohlendioxidemissionen verwendet werden, um Methanol zu erzeugen. Dieses Methanol dient dann gleichzeitig wieder als Energiespeicher, ist leicht zu transportieren und kann insbesondere, je nach Bedarf, wieder eingesetzt werden. Durch das Verfahren wird damit ein Handelsprodukt, was in hohem Maße benötigt wird, zur Verfügung gestellt, was auch mit relativ wenig Aufwand an andere Einsatzorte zu transportieren ist.

Ein Problem im Stand der Technik ist es derzeit, dass die Verwendung von Elektroenergie aus Windenergieanlagen es erfordert, dass zur Gewährleistung der Netzsicherheit Einrichtungen zu installieren sind, mit deren Hilfe die Leistung der Windenergieanlagen in Schwachlast- und Starkwindperioden im Rahmen eines Netzsicherheits-Managements erforderlichenfalls gedrosselt werden kann. Es ist weiterhin nachteilig, dass die "Windenergieernte" durch Schwachlast im Netz bei Starkwind, vor allem an Sonn- und Feiertagen, sowie nachts, trotz des Vorrangs bei der Einspeisung, insofern beeinträchtigt wird, als dem konventionellen Kraftwerkspark eine geringe Restlast verbleiben muss, die zum gehäuften An- und Abfahren und zu Teillastbetrieb mit erhöhten spezifischen Verbrauchswerten und zur Erhöhung von CO2-Emissionen führt.

Bei dem geplanten Aufkommen an Elektroenergie aus sogenannten Off-Shore-Windenergieanlagen wird im Vergleich zum heutigen Stand durch die hohe Einspeiseleistung von Off-Shore-Windenergieanlagen-Clustem eine neue Qualität bei den Anforderungen zur Auslastung der Netze erreicht, die zu erheblichen Effektivitätsverlusten bei der Nutzung regenerativer Energien führt und insbesondere die Gefahr besteht, dass die Netze "überspeist" werden, wodurch die Drosselung der Einspeisungsleistung erforderlich ist. Dies wird durch das jetzt zur Verfügung gestellte Verfahren beseitigt, indem jetzt in Spitzenzeiten, in welchen ein hohes Maß "überschüssiger" Elektroenergie zur Verfügung steht, aus den Off-Shore-Anlagen am Ort des Entstehens, nämlich in der Nähe des traditionellen, fossil befeuerten Kraftwerkes eine Elektrolyseanlage zur Spaltung von Wasser in Wasserstoff und Sauerstoff installiert wird und die überschüssige Energie diesbezüglich eingesetzt wird. Der Wasserstoff wird dann entweder vor Ort gespeichert, was nicht so ein hohes Gefahrenpotential birgt als dessen Transport, oder aber gleich einer Methanolsynthese zugeführt, indem das Kohlendioxid aus dem fossil befeuerten Kraftwerk oder aber aus einem Speicher gezogen wird, dies mit dem Wasserstoff gemischt und unter Verwendung von Katalysatoren zu Methanol umgewandelt wird.

Das erfindungsgemäß erzeugte Methanol stellt demnach sowohl ein Verkaufsprodukt als auch gleichzeitig einen Elektroenergiespeicher dar, nämlich den Elektroenergiespeicher für die regenerativ gewonnene Elektroenergie. Dieser Energiespeicher kann dann im Bedarfsfall im fossil befeuerten Kraftwerk dem Verbrennungsprozess wieder zugeführt werden, wenn beispielsweise nicht ausreichend regenerative Elektroenergie zur Verfügung steht. Im Weiteren ist zu erwarten, dass aufgrund der hohen Einspeisung von Strom aus regenerativen Energiequellen in allen Netzebenen das ganze Jahr über zu jeder Zeit in einer angenommenen Regelzone, beispielsweise Deutschland, durch den Ausgleich in den vorhandenen Regelzonen immer regenerativ generierter Strom mit einer Leistung von schätzungsweise ca. 500 MW vorhanden ist. Dieser Strom kann ebenfalls zur Erzeugung von Wasserstoff eingesetzt werden, um diesen dann wieder mit Kohlendioxid zu Methanol zu verarbeiten. Der Vorteil der gesamten Verfahrensweise ist auch der, dass der überschüssige Sauerstoff ebenfalls vor Ort, ohne hohe Transportaufwendungen, einsetzbar ist.

Das Verfahren verwendet, wie vorher bereits umrissen, Elektroenergie, bevorzugt aus umweltfreundlichen Energieerzeugungsanlagen, insbesondere aus Energieerzeugungsanlagen für erneuerbare Energie zur Herstellung des für die Methanolsynthese benötigten Wasserstoffs.

Dies wird dadurch erreicht, dass die bei der Elektrolyse benötigte Elektroenergie aus dem Überschussaufkommen von Wind- und/oder Solarenergieerzeugungsanlagen, insbesondere in Schwachlastphasen von Elektroenergienetzen zur Verfügung gestellt und verwendet wird. Das Verfahren ist demnach durchführbar, wenn zu viel Wind-und/oder Solarenergie zur Verfügung steht oder aber in Zeiten, in denen wenig Elektroenergie abgenommen wird (Schwachlastphasen). Damit ist natürlich ein universeller Einsatz mit Hilfe von entsprechenden Steuermechanismen problemlos jetzt möglich.

Für das Verfahren wird mit Hilfe der Elektrolyse Wasser in Wasserstoff und Sauerstoff gespalten, diese anschließend gegebenenfalls gespeichert und dem Energieerzeugungsprozess zumindest teilweise in Form des Sauerstoffs wieder zugeführt werden. Der Wasserstoff wird zur Methanolherstellung genutzt, so dass ein geschlossener Prozess mit absolut geringen Umweltemissionen entsteht.

Für die Elektrolyse wird bevorzugt mit Hilfe von Wind- und/oder Solarenergieanlagen gewonnene, insbesondere bevorzugt überschüssige Elektroenergie aus Starkwind- und/oder Schwachlastperioden zur Herstellung von Windwasserstoff verwendet, der mit dem aus dem Verbrennungsprozess fossiler Energieträger aus Kraft- oder Heizkraftwerken, sowie aus Anlagen der Zement-, Chemie-, Hüttenindustrie beziehungsweise in Biogasanlagen gewonnenen Kohlendioxid gemischt wird und zu Methanol, unter Verwendung von Katalysatoren, umgesetzt wird. Die Verfahren zur Herstellung von Methanol aus Kohlendioxid, unter Verwendung von Wasserstoff, sind dabei im Stand der Technik hinlänglich bekannt und können hier allerdings vor Ort, nämlich an der Elektroenergieerzeugungsanlage oder aber in der Nähe eines entsprechenden Speichers für CO2 installiert werden, so dass es nunmehr möglich ist, die bisher die Umwelt belastenden Abgase zu verringern und das Kohlendioxid sinnvoll einzusetzen.

Das Kohlendioxid wird aus dem Abgas der Kraft- oder Heizkraftwerke sowie aus den Anlagen der Zement-, Chemie-, Hüttenindustrie beziehungsweise in Biogasanlagen der Methanolsynthese direkt zugeführt. Das heißt, das Kohlendioxid wird direkt aus dem Abgas adsorbiert oder absorbiert und dann der Methanolsynthese direkt zugeführt. Dort wird es mit dem zur Verfügung stehenden Wasserstoff gemischt und unter Verwendung der Katalysatoren zu Methanol umgesetzt.

Das Verfahren, wie vorher beschrieben, lässt sich allerdings auch so durchführen, dass das Kohlendioxid aus Speichern beziehungsweise Speicheranlagen der Methanolsynthese sozusagen indirekt zugeführt wird. Auch diese Ausgestaltung ist von der Erfindung umfasst.

Wie bereits recht ausführlich beschrieben wird das erzeugte Methanol bevorzugt in einem Methanollager zwischengespeichert, bevor es beispielsweise dem Feuerungsprozess wieder zugeführt oder aber abgefüllt wird.

Das erzeugte Methanol kann im Transportbehälter und/oder Transportbeziehungsweise Verkaufsgebinde abgefüllt werden. Mit diesen Transportbeziehungsweise Verkaufsgebinden lässt sich das Methanol an jeden gewünschten Ort transportieren.

Ebenso kann das erzeugte Methanol in wenigstens einer Pipeline zu wenigstens einem Heizkraftwerk transportiert wird.

Die technologische Einheit der Kohlendioxid-Verwertung und Wasserstoff-Methanolspeicherung (CV WMS) wird containerisiert am Standort einer Kohlendioxid-Quelle eingesetzt beziehungsweise installiert wird. Durch eine umsetzbare technologische Einheit der Kohlendioxid-Verwertung ist es jetzt möglich, am Standort der CO2-Quelle, egal ob es sich dabei um ein konventionelles Kraftwerk handelt oder um einen CO2-Speicher, dort Methanol zu erzeugen. Das Methanol kann dann entweder ebenfalls in einen Methanol-Speicher oder aber über eine Abfüllanlage abgefüllt und dann wegtransportiert werden.

Regenerativ gewonnene Elektroenergie, die bei der gegenwärtig erreichten hohen Durchdringung in einer Regelzone verfügbar ist, wird vorrangig im 20 kV-Netz von Heiz-, Kraft- oder Heizkraftwerken, Biogasanlagen und anderen Kohlendioxid-Emittenten zum Schutze des Netzes dem internen Verbraucher, nämlich der Kohlendioxid-Verwertungs- und Wasserstoff-Methanol-Speichereinheit zugeführt und als Maßnahme im Rahmen des Netzsicherungs-Managements zur Netzstabilisierung eingesetzt beziehungsweise verwendet. Dadurch wird eine Entlastung der Netze insbesondere dann erreicht, wenn bestimmte Energiespitzen zur Verfügung stehen.

Bei einer wärmegeführten Betriebsweise des Heiz-, Kraft- oder Heizkraftwerkes erfolgt eine Reduzierung der Kohlendioxid-Emissionen durch die Kohlendioxid-Verwertung zu Methanol mit selbst erzeugtem Strom, der an sich als negative Regelleistung vorgehalten wird, wobei der im Kraftwerk hergestellte Strom ebenfalls zur Kohlendioxid-Verwertung eingesetzt werden kann, um eine durch die vorrangige Einspeisung von erneuerbaren Energien bedingte Teillastfahrweise mit deutlich schlechteren spezifischen Verbrauchswerten und erhöhten Kohlendioxid-Emissionsraten beziehungsweise die Gefahr von Häufungen bei An- und Abfahrten des beziehungsweise der Kraftwerke(s) zu überwinden. Demnach gelingt es durch das erfindungsgemäße Verfahren nicht nur, die umweltschädlichen Emissionen der traditionellen mit fossilen Brennstoffen arbeitenden Kraftwerke zu reduzieren, sondern gleichzeitig auch die Netze von entsprechenden Energiespitzen zu befreien. Des Weiteren können die mit fossilen Brennstoffen betriebenen Kraftwerke zumindest in einem Bereich gefahren werden, bei dem eine Effizienz weiterhin gegeben ist und gleichzeitig ein geringer Schadstoffausstoß.

Die Kohlendioxid-Verwertungs- und Wasserstoff-Methanol-Speichereinheit befindet sich im heiz-und kraftwerks- beziehungsweise industrienahen Bereich beziehungsweise nahe einer Biogasanlage, die insbesondere mit Abfallwasserstoff betrieben wird. So werden Synergieeffekte hinsichtlich der Ver- und Entsorgung von Betriebs- und Hilfsmitteln sowie hinsichtlich des Austausches von Wärme- und Kälteenergie optimal genutzt.

Von Vorteil ist es auch, dass das Kohlendioxid für den Wasserstoff als Transporteur fungiert und damit die CO2-Power-Carrier-Technologie (CPCT) begründet, die das Verfahren zur Verwertung von Kohlendioxid mit Hilfe von Wasserstoff vorzugsweise aus erneuerbaren Energien zu Methanol zum Inhalt hat und die im Ergebnis dieses Verfahrens CO2-Emissions-Zertifikate freisetzt. Das Freisetzen von sogenannten Kohlendioxid-Emissions-Zertifikaten ist ein großer Vorteil, nicht nur derart, dass mit diesen Zertifikaten gehandelt wird. Vielmehr ist es durch das erfindungsgemäße Verfahren jetzt gegeben, dass der Anteil von Kohlendioxid, mit dem die Umwelt belastet wird, tatsächlich und nicht nur ideell reduziert wird. Gleichzeitig kann man allerdings den ökonomischen Effekt der CO2-Emissions-Zertifikate nutzen, um die eigene Effizienz weiter zu erhöhen.

Das hergestellte Methanol wird in den Feuerungsprozess von Kraftwerken zur Stromerzeugung zurückgeführt, um Windflauten auszugleichen und das dabei freiwerdende Kohlendioxid erneut zur Synthese von Methanol einzusetzen. Demnach ist ein Kreislauf geschaffen, mit dem es gelingt, die Kohlendioxid-Belastung durch ein konventionelles, mit fossilen Heizmaterialien betriebenes Kraftwerk nahezu vollständig zu reduzieren.

Wie bereits weiter vorn erwähnt, wird Windelektroenergie aus Starkwind- und Schwachlast-Perioden, die insbesondere an den Wochenenden und an Feiertagen sowie nachts auftreten, dazu verwendet, die Verfahren, wie vorher beschrieben, durchzuführen. Dabei wird diese Wind-Elektroenergie insbesondere für die Elektrolyse von Wasser zu Wasserstoff und Sauerstoff eingesetzt. Auch damit wird die gesamte Effizienz der Anlage erhöht. Durch die Möglichkeit der Speicherung des zur Verfügung stehenden Kohlendioxids und Wasserstoffs in Form von Methanol ist auch eine effizientere Fahrweise des gesamten Energieerzeugungskomplexes jetzt möglich.

Die Verfahrensschritte Elektrolyse, Kohlendioxidad- beziehungsweise - absorption und Methanolsynthese werden derart gestaltet, dass durch die Entkopplung eine weitgehend reduzierte Fahrweise des/der Kraftwerke(s) regel- und maschinentechnisch gewährleistet wird.

Es wird die Erzeugung von Elektroenergie zur Verfügung gestellt, wobei insbesondere das gewonnene Methanol als Wärmeerzeugungsträger zumindest als Zusatzwärmeerzeugungsträger zu fossilen Wärmeerzeugungsträgem dem Verbrennungsprozess von konventionellen, mit fossilen Heizkraftstoffen betriebenen Kraftwerken, insbesondere in Starklastphasen beziehungsweise bei nicht ausreichend zur Verfügung stehender Elektroenergie aus Schwachwindphasen eingesetzt wird. Dadurch wird deutlich, dass das in dem vorher beschriebenen Verfahren zur Erzeugung von Methanol jetzt vorhandene Methanol tatsächlich zur Abpufferung von Spitzen des Energiebedarfs eingesetzt werden kann. Insbesondere kann das Methanol auch dann eingesetzt werden, wenn nicht ausreichend Elektroenergie aus Windkraftwerken beziehungsweise Solarenergieerzeugungsanlagen zur Verfügung steht.

Nachfolgend wird die Erfindung nochmals kurz schwerpunktmäßig zusammengefasst und erläutert:
Die Erfindung betrifft den Verfahrensweg zur Verwertung von Kohlendioxid aus dem Abgas fossil befeuerter Heizkraftwerke und anderer CO2 - Emittenten wie der Zement-, Chemie-, Stahl- usw. Industrie und auch aus Biogasanlagen mit Hilfe von Wasserstoff, der durch Elektrolyse von Wasser mit Hilfe von elektrischem Strom aus regenerativen Energiequellen gewonnen wurde, zur Herstellung von Methanol.
Der Verfahrensweg dient gleichzeitig zur Speicherung Erneuerbarer Energien in Form von Methanol.

Insbesondere im 20 kV-Netz werden Lasten geschaffen, um das mit Hilfe von Windenergieanlagen oder anderen regenerativen Energiequellen gewonnene Elektroenergieaufkommen insbesondere von Offshore-Windenergieanlagen-Clustern bei Starkwind, das auf Grund bereits bestehender hoher Kapazitätsbelastungen der Netze aller Ebenen oder einzelner Übertragungsnetze bei Schwachlast durch das Netzsicherheitsmanagement zur Netzstabilisierung abgeboten werden müsste, in Wasserstoff umzuwandeln und mit Kohlendioxid zur Synthese von Methanol bzw. Kohlenwasserstoffen umzusetzen.

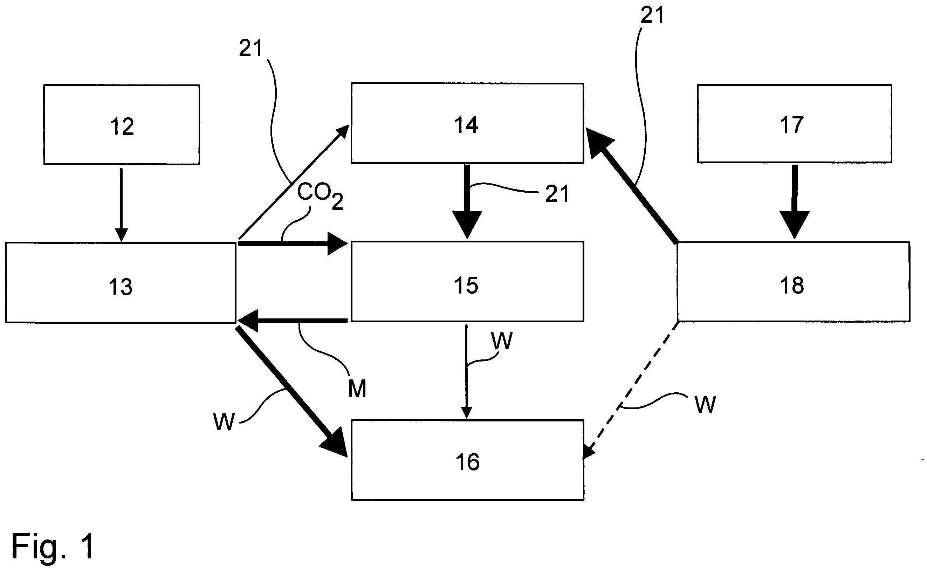

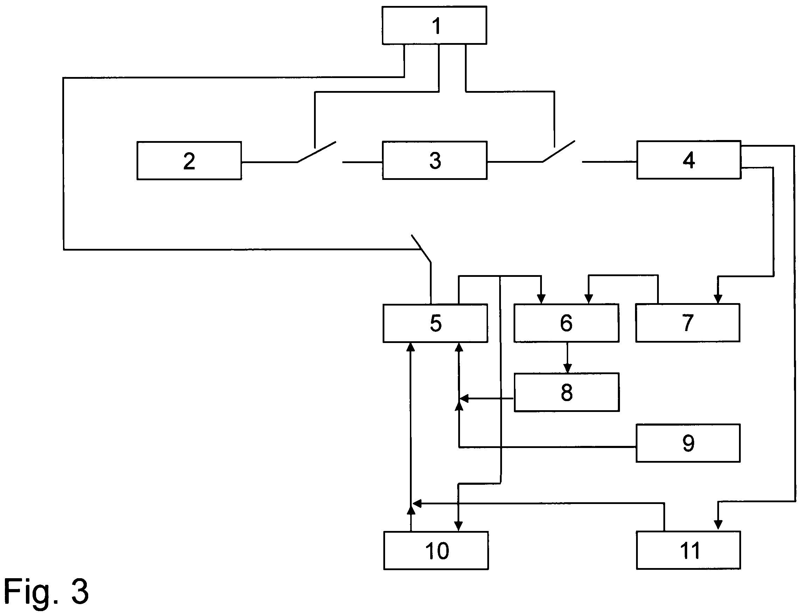

Es werden Lasten durch CO2-Verwertungs- und Wasserstoff-Methanol-Speichereinheiten (CV WMS) gebildet, die durch die Elektrolyse (Fig. 3) zur Herstellung von Wasserstoff und Sauerstoff zur Abnahme von Elektroenergie aus dem Netz und damit zur Netzentlastung führen. Zu dieser technologischen Einheit gehören weiterhin die CO2- Ad - bzw. Absorptionsanlage zur CO2-Bereitstellung sowie die Methanolanlage zur Herstellung von Methanol. (Fig. 1-3).

Es ist insbesondere die Möglichkeit beinhaltet, sog. "überschüssige" Windelektroenergie aus Starkwind- und Schwachlastperioden durch wirksame Kommunikationswege zwischen dem Netzsicherheitsmanagement und der Last im 20 kV-Netz unmittelbar für die Methanolsynthese zu nutzen.

Bei der CO2-Verwertungs- und Wasserstoff-Methanol-Speichereinheit (CV WMS) wird auf an sich bekannte und langjährig erprobte Verfahren zurückgegriffen, die auf neuartige und verbesserte Art und Weise kombiniert und containerisiert installiert werden kann.

In dem Umfang, in dem nach dem gefundenen Verfahrensweg Methanol hergestellt wird, wird eine äquivalente Menge fossiler Energieträger und damit die adäquate CO2-Emission sowie CO2 -Emissions-Zertifikate eingespart.

Die Lösung besteht nun vorrangig darin, durch Verknüpfung der sich auf dem Energiesektor abzeichnenden Entwicklungen einen Verfahrensweg gefunden zu haben, um klimaschädliches CO2-Emissionen aus der Energieerzeugung durch Verwertung zu dem Industrieprodukt Methanol zu reduzieren, eine Möglichkeit aufzuzeigen, um im Untergrund gespeicherte CO2-Mengen langfristig abzubauen, regenerativ gewonnene Elektroenergie über Wasserstoff zu speichern, so genannte überschüssige Elektroenergie insbesondere aus Repowering von WEA und Offshore-Windenergieanlagen-Clustern effektiv zu nutzen und Maßnahmen zur Sicherung der Netzverträglichkeit hoher Windstromeinspeiseleistungen durch Netzentlastung mit Hilfe beeinflussbarer Lasten in Form der Methanolproduktion zu schaffen.

Mit CO2-Verwertungs- und Wasserstoff-Methanol-Speichereinheiten (CV WMS) werden elektrische Lasten und generell Möglichkeiten geschaffen, auch Abfall-Wasserstoff insbesondere aus chemischen Prozessen in ein gängiges Industrieprodukt wie Methanol umzusetzen und stoffwirtschaftlich oder energetisch zu nutzen.

Das CO2 wird zum Wasserstoff-Energietransporteur und bildet damit die Basis für die CO2-PowerCarrier-Technologie (CPCT).

Unter Anwendung der CO2-PowerCarrier-Technologie (CPCT) können Kraft- oder Heizkraftwerke in Starkwindperioden, die durch einen hohen spezifischen Wärmeenergiebedarf bei gleichzeitig geringer Leistungsabnahme gekennzeichnet sind, in den Standby-Betrieb oder auch Warmhaltebetrieb übergehen, der dann durch das Verfeuern von selbst hergestelltem Methanol nahezu emissionsfrei erfolgt.

Einer der Grundgedanken besteht darin, durch entsprechende verfahrenstechnische Verknüpfungen der Produktionsströme die naturgegebenen Unregelmäßigkeiten beim Aufkommen von Wind- bzw. Solarstrom durch Verstromung von Methanol, d.h. Rückführung des Endproduktes Methanol in den Feuerungsbereich des Heiz-, Kraft- oder Heizkraftwerks und Beginn eines neuen Zyklus der Methanolproduktion mit Zwischenspeicherung von Kohlendioxid, weitgehend zu nivellieren.

Ein weiterer Vorteil bei der Nutzung des vorgeschlagenen Verfahrensweges wird darin gesehen, das betriebswirtschaftliche Ergebnis eines Heiz-, Kraft- oder Heizkraftwerks durch den Verkauf nicht genutzter CO2-Emissionszertifikate zu verbessern.

Mit dem vorgeschlagenen Verfahrensweg ist bei einer wärmegeführten Betriebsweise des Heiz-, Kraft- oder Heizkraftwerks eine Reduzierung der CO2-Emissionen durch CO2-Verwertung zu Methanol mit selbst erzeugtem Strom, der an sich als negative Regelreserve für hohe Windenergieeinspeisungen vorgehalten wird, möglich.

Selbst erzeugter Strom kann ebenfalls in einer CV WMS - Einheit eingesetzt werden, um Teillastfahrweisen des Heiz-, Kraft- oder Heizkraftwerks zu verlassen, bei denen infolge einer Unterlastfahrweise deutlich schlechtere CO2-Emissionsraten resultieren. Es kann ebenfalls die Gefahr gehäufter Abfahr- und Startvorgänge reduziert werden.

Mit dem vorgeschlagenen Verfahrensweg wird vor allem die Möglichkeit geschaffen, regenerative Energien wie Windstrom in ein Handelsprodukt umzuwandeln, dessen Anwendungsinfrastruktur seit Jahren erprobt ist und das einen wichtigen Synthesebaustein der C1-Chemie darstellt, der weitgehend in die Carbochemie integriert werden kann.

Ein weiterer Vorteil des erfindungsgemäßen Verfahrensweges wird darin gesehen, dass der bei der Elektrolyse mit Hilfe regenerativ gewonnener Elektroenergie erzeugte Sauerstoff komplett in modernen Kraftwerkstechnologien wie dem Oxyfuel-Prozess zur Verstromung von Kohle eingesetzt werden kann.

Reaktionsgleichungen des vorgeschlagenen Verfahrensweges:

Elektrolyse:

H2O + Elt → H2 + 1/2 02

Methanolsynthese:

CO2 + H2 → CO + H2O + ΔH (+ 41,20 kJ / Mol)

CO + 2 H2 → CH3OH - ΔH (- 90,84 kJ / Mol)

CO2 + 3 H2 → CH3OH + H2O - ΔH (- 49,64 kJ / Mol)

Es wird eine Netzentlastung großer Elektroenergienetze zur Verfügung gestellt, welches sich dadurch auszeichnet, dass Windelektroenergie, die in Starkwind- und Schwachlast-Perioden zu einem instabilen Netzsicherheitszustand in allen verfügbaren elektrischen Netzebenen führen kann und abgeboten werden müsste über die Elektrolyse in das Verfahren der Kohlendioxid-Power-Carrier-Technologie zur Verwertung von Kohlendioxid zur Speicherung von erneuerbaren Energien in Form von Methanol als beeinflussbare Last eingeführt wird.

Die Erfindung betrifft auch eine Energieerzeugungsanlage mit wenigstens einem mit fossilen Wärmeerzeugungsträger befeuerbaren Heizkraftwerk, wenigstens einer Dispatcher- oder Verteilerstation, wenigstens einem mit Wind- und/oder Wasserkraft betriebenen Kraftwerk und/oder einem Solarkraftwerk, wenigstens einer Elektrolyseanlage zur Spaltung von Wasser in Wasserstoff und Sauerstoff, wenigstens je einer Speicherstation für Kohlendioxid, Sauerstoff und Wasserstoff sowie wenigstens einer Methanolerzeugungsanlage zur Durchführung einer Methanolsynthese, wenigstens einen Methanolspeicher sowie einer Verknüpfung oder Vernetzung der einzelnen Anlagenbestandteile und einer Steuerung, die die Anlagenbestandteile zur Energieerzeugung in Abhängigkeit vom Strombedarf zur Optimierung der Auslastung zu steuern vermag, wobei die einzelnen Anlagenbestandteile an einem einzigen Standort vorgesehen sind. Erfindungsgemäß ist eine technologische Einheit einer CO2-Verwertung und Wasserstoff - Methanol - Speicherung (CV WMS) als eine containerisierte Einheit angeordnet, und diese besteht aus
- einer Elektrolyse von Wasser mit H2 - und O2 -Gewinnung,
- einer CO2 - Ad- oder Absorption mit CO2 - Gewinnung und
- einer Methanolsynthese mit MeOH - Speicherung. Die Anlagenteile der technologischen Einheit der CO2-Verwertung und Wasserstoff-Methanol-Speicherung (CV WMS) sind in unterschiedlichen Modulen angeordnet, die durch Schnittstellen miteinander verbindbar sind, und die Anlagenteile der technologischen Einheit der CO2-Verwertung und Wasserstoff-Methanol-Speicherung (CV WMS) sind in wenigstens einem Container angeordnet.

Damit wird eine Energieerzeugungsanlage, die insbesondere auch aus einer Verknüpfung von unterschiedlichen Energieerzeugungsträgem, wie z. B. Heizkraftwerken, Wasserkraftwerken, Solarkraftwerken oder Windkraftwerken besteht, zu optimieren und insbesondere die derzeit noch auftretenden Energiespitzen bei Starkwindphasen und geringem Energiebedarf beziehungsweise bei Schwachwindphasen und hohem Energiebedarf optimal auszugleichen. Es gelingt, die aus umweltfreundlichen, regenerativen Energieerzeugungsanlagen erhaltene Elektroenergie in Form von Wasserstoff und Methanol zwischenzuspeichern und insbesondere auch den überschüssigen Sauerstoff einer optimalen Verwertung in der Energieerzeugungsanlage wieder zuzuführen.

Die einzelnen Anlagenbestandteile sind an einem einzigen Standort, also territorial gebündelt, vorgesehen. Dies hat den Vorteil, dass geringe Transportwege zu überwinden sind.

Die technologische Einheit der Kohlendioxidverwertung und Wasserstoff-Methanol-Speicherung besteht aus
Elektrolyse von Wasser mit Wasserstoff- und Sauerstoffgewinnungen Kohlendioxid-Ad- beziehungsweise Absorption mit Kohlendioxidgewinnung und
Methanolsynthese mit Methanolspeicherung. Diese technologische Einheit kann dann an dem geeignetsten Standort installiert werden oder sie ist als umsetzbare Einheit ausgebildet.

Dabei sind alle Anlagenteile in einer einzigen umsetzbaren Einheit vorgesehen. Es ist allerdings auch von Vorteil, wenn die technologische Einheit der Kohlendioxidverwertung und Wasserstoff-Methanol-Speicherung in unterschiedlichen Modulen vorgesehen sind, wobei die einzelnen Anlagenmodule durch Schnittstellen miteinander verbindbar sind.

Die Energieerzeugungsanlage zeichnet sich auch dadurch aus, dass die technologische Einheit beziehungsweise die einzelnen Anlagenmodule in einem Container angeordnet beziehungsweise vorgesehen sind.

Die Erfindung wird nachfolgend anhand von Ausführungsbeispielen weiter beschrieben. Es zeigen:

Fig. 1 : eine schematische Darstellung der Kohlendioxid-Power-Carrier-Technologie als Bestandteil des erfindungsgemäßen Verfahrens
Fig. 2 : die verfahrenstechnischen Bestandteile der Kohlendioxid-Power-Carrier-Technologie und deren Zusammenwirken
Fig. 3 : Fließschema zum vorgeschlagenen Verfahrensweg zur Herstellung von Methanol, zur Speicherung von Elektroenergie und insbesondere eine schematische Darstellung der erfindungsgemäßen Elektroenergieerzeugungsanlage;

Fig. 1 zeigt eine schematische Darstellung der Kohlendioxid-Power-Carrier-Technologie als Bestandteil des erfindungsgemäßen Verfahrens. Wie aus der schematischen Darstellung ersichtlich, wird Kohle, Erd- oder Biogas als fossiler Energieträger als Ausgangsprodukt eingesetzt. Dies ist schematisch in dem Rechteck mit dem Bezugszeichen 12 bezeichnet. Diese fossilen Energieträger werden in einem Kraft- und/oder Heizwerk, welches mit dem Bezugszeichen 13 bezeichnet ist, in Strom 21 umgewandelt. Dieser Strom 21 wird in das Nieder-, Mittel- oder Hochspannungsnetz 14 eingespeist. In das Nieder-, Mittel- oder Hochspannungsnetz 14 wird auch Strom aus Wind- und/oder Photovoltaikanlagen oder auch aus Wasserkraftwerken eingespeist, die schematisch mit dem Bezugszeichen 18 als umweltfreundliche Energieerzeugungsanlagen bezeichnet sind. Mit 17 wird Wind-, Wasserkraft und/oder die Solarstrahlung schematisch bezeichnet. Der Strom aus dem Nieder-, Mittel- oder Hochspannungsnetz 14 wird, wie aus dem Pfeil ersichtlich, in der CO2-Power-Carrier-Anlage 15 verarbeitet. Dazu wird einerseits Rauchgas aus dem Kraft- und/oder Heizwerk 13 der CO2-Verwertungs- und Wasserstoff-Methanol-Speichereinheit (CV-WMS) zugeführt. Des Weiteren ist es selbstverständlich notwendig, hier Wasserstoff zu erzeugen. Da die Methanolerzeugung ein endogener Prozess ist, entsteht Wärme W. Diese Wärme und die Abwärme aus dem Kraft- und/oder Heizwerk 13 wird einem nicht näher bezeichneten Wärmeverbraucher 16 zugeführt. Des Weiteren wird auch die Abwärme aus der CO2-Verwertungs- und Wasserstoff-Methanol-Speichereinheit (CV-WMS) 15 dem Wärmeverbraucher 16 zugeführt. Gleiches gilt für die Wärme, die aus Energie aus dem mit 18 bezeichneten Bereich erzeugt wird. Damit wird eine optimale Ausnutzung der fossilen Energieträger zum einen und zum anderen eine optimale Ausnutzung der Wind- und/oder Solarstrahlung beziehungsweise der Wasserkraft erreicht. Über geeignete Steuerungen lässt sich die gesamte Anlage so steuern, dass erfindungsgemäß bevorzugt Elektroenergie aus Wind- und/oder Photovoltaikanlagen beziehungsweise aus Wasserkraftwerken der CO2-Verwertungs- und Wasserstoff-Methanol-Speichereinheit (CV-WMS) zugeführt wird, insbesondere um den nötigen Wasserstoff durch Elektrolyse zu erhalten. Die Fig. 1 beschreibt demnach die zentrale Stellung der CO2-Power-Carrier-Technologie (CPCT) und die CV-WMS-Einheit mit den Stoff- und Energieströmen sowie deren Integration in die Elektronetzebenen, das Kraft- und/oder Heizwerk beziehungsweise die Biogasanlage, die erneuerbaren Energiequellen und die Wärmeverbraucher. Dies ist insgesamt ein geschlossener Kreislauf, der eine optimale Auslastung der eingesetzten fossilen Energieträger und gleichzeitig auch der durch die erneuerbaren Energiequellen zur Verfügung gestellten Elektroenergie dar.

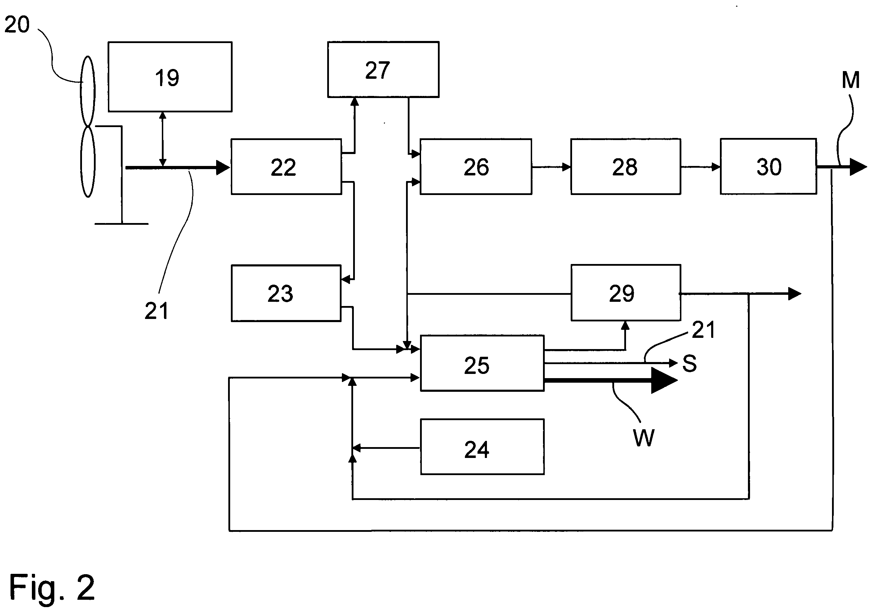

In Fig. 2 sind die verfahrenstechnischen Bestandteile der CO2-Power-Carrier-Technologie und deren Zusammenwirkung dargestellt. Mit dem Bezugszeichen 20 ist schematisch eine Windenergieanlage (WEA) dargestellt, welches Strom 21 entweder in das Nieder-, Mittel- oder Hochspannungsnetz 19 einspeist oder aber der Elektrolyse 22 zuführt. Aus der Elektrolyse 22 wird Wasserstoff in einem Wasserstoffspeicher 27 zugeführt. Des Weiteren wird Sauerstoff einem Sauerstoffspeicher 23 aus der Elektrolyse zugeführt. Der Wasserstoff aus dem Wasserstoffspeicher 27 gelangt in einen Synthesegasverdichter 26 und von dort in die Methanolsynthese 28. Das erzeugte Methanol gelangt in einen Methanolspeicher 30 und kann von dort entweder abgefüllt oder dem Kraft- und/oder Heizwerk 25 wieder zugeführt werden. Die Einspeisung mit fossilen Energieträgern, wie Kohle, Erz-oder Biogas trägt das Bezugszeichen 24. Aus dem Kraft- und/oder Heizwerk 25 gelangt CO2 in den CO2-Speicher 29, welches, wie hier noch nicht dargestellt, erfindungsgemäß dann der Methanolsynthese zumindest teilweise zugeführt wird. Teilweise wird das CO2 allerdings auch wieder dem Kraft- und/oder Heizwerk 25 zugeführt. Dies ist wesentlicher Bestandteil der Oxyfuel-Technologie. Mit dem Bezugszeichen 23 ist noch ein Sauerstoffspeicher dargestellt, der den in der Elektrolyse erzeugten Sauerstoff zu speichern vermag und der ihn dann dem Kraft-und/oder Heizwerk 25 wieder zuführt, um bei der Verbrennung der fossilen Energieträger eine optimale Leistung zum einen zu gewährleisten und zum anderen ein sehr reines CO2 zu erhalten, welches gut speicherbar ist. Mit dem Bezugszeichen 21 ist wieder der erzeugte Strom bezeichnet. Der Pfeil mit dem Bezugszeichen W bezeichnet die abgeführte Wärme. Die Abbildung 2 stellt hier allerdings nicht den gesamten erfindungsgemäßen Komplex dar, sondern nur ein Detail, nämlich den immanenten Bestandteil der CO2-Power-Carrier-Technologie am erfindungsgemäßen Verfahren.

In der Fig. 3 ist ein Fließschema zum vorgeschlagenen Verfahrensweg nach der Erfindung zur Herstellung von Methanol, zur Speicherung von Elektroenergie und insbesondere eine schematische Darstellung der erfindungsgemäßen Elektroenergieerzeugungsanlage dargestellt. In der Netzleitstelle 1 der entsprechenden Regelzone erfolgt die Kontrolle und Regelung der Lastflüsse zur Einhaltung der Stabilität der Netze 3 und insbesondere zur Integration des Aufkommens aus erneuerbaren Energiequellen, wie Windenergieanlagen 2. Die Elektrolyse 4 bezieht ihren Strombedarf vorrangig zur Netzentlastung aus dem Netz, in das jedoch auch Strom aus dem Kraftwerk 5 und anderen Quellen eingespeist wird. Die Elektrolyse 4 produziert sowohl Wasserstoff, der in einem Wasserstoffspeicher 7 eingespeist wird, als auch Sauerstoff, der in einem Sauerstoffspeicher 11 zwischengelagert wird. Aus dem Heizwerk oder Heizkraftwerk 5, das mit fossilen Energieträgern beziehungsweise Biogas aus dem Erd- beziehungsweise Biogas oder Kohlespeicher 9 versorgt wird, werden CO2-haltige Rauchgase imitiert, aus denen das Kohlendioxid in einer Ad- beziehungsweise Absorptionsanlage gewonnen und in einem CO2-Speicher 10 zwischengelagert wird. Wasserstoff und CO2 werden vermischt und über einen Verdichter in das Kreislaufgas der Methanolanlage 6 mit dem erforderlichen Betriebsdruck eingespeist. Das hergestellte Methanol wird dem Methanoltanklager 8 zugeführt. Wie ersichtlich, ist damit die erfindungsgemäße Erzeugung von Methanol zum einen dargestellt. Zum anderen ist jedoch auch ersichtlich, dass hier eine optimale Verwertung der aus erneuerbaren Energiequellen zur Verfügung gestellten Elektroenergie erfolgt. Des Weiteren wird deutlich, dass es mit dem erfindungsgemäßen Verfahren jetzt gelingt, in Phasen, in denen überschüssig Elektroenergie erzeugt wird und gleichzeitig die Abnahme sehr gering ist, diese Elektroenergie sinnvoll in Form von Methanol zu speichern. In dem Fall, wenn beispielsweise nicht ausreichende Wind- oder Wasserenergie oder Solarenergie zur Verfügung steht und gleichzeitig der Bedarf an Elektroenergie steigt, kann dann dieses Methanol wieder sinnvoll eingesetzt werden, indem es dann dem Heiz- oder Heizkraftwerk 5 wieder zugeführt wird. Insgesamt wird damit selbstverständlich auch eine bedeutende Reduzierung der Umweltbelastung durch schädliches CO2 erreicht, weil dieses fast vollständig im Kreislauf gebunden wird und sinnvoll der Methanolerzeugung zugeführt werden kann.

### Ausführungsbeispiel:

Für die Realisierung des vorgeschlagenen Verfahrensweges zur Reduzierung von CO2 - Emissionen durch Verwertung des Kohlendioxid mit Hilfe von Wasserstoff, der durch Elektrolyse von Wasser mit regenerativ gewonnener Elektroenergie, vorzugsweise Strom aus Windkraftanlagen, zu Methanol umgesetzt wird und in den Abb. 1 - 3 dargestellt ist, bietet sich beispielhaft Lubmin bei Greifswald in Mecklenburg-Vorpommern als Modell-Standort an.

Für diesen Standort spricht ebenfalls die Nähe der Zentren für das Netzsicherheitsmanagement und die relativ hohe Dichte an Wärmebedarf und CO2-Aufkommen in der Region entlang der Ostsee-Küste.

Negativ ist das Fehlen großer elektrischer Lasten in dem dünn besiedelten und industriell relativ wenig erschlossenen Gebiet zu beurteilen. Weitere Beispiele sind denkbar.

An diesem Standort ist sowohl die Anlandung der fossilen Energieträger Steinkohle als auch Erdgas und deren Verstromung geplant. Vorgesehen ist die Errichtung leistungsstarker Kraftwerksanlagen auf Basis dieser fossilen Energieträger, die unterschiedlich für die Einspeisung in den Grundlastbereich bzw. in den Mittel- und Spitzenlastbereich ausgelegt werden sollen. Am gleichen Standort ist ebenfalls die Einschleifung der Elektroenergie aus leistungsstarken Offshore-Windparks um Rügen mit Gesamtleistungen von >2.000 MW vorgesehen.

Aus dieser Situation heraus sind in Starkwind- und Schwachlastperioden große Lastflüsse in Nord-Süd-Richtung und durchaus Überspeisungen der vorhandenen Netze zu erwarten.

Aus geologischer Sicht ist der Standort Lubmin durch die Nähe ausgebeuteter, fachmännisch versiegelter Erdgas- bzw. Erdöl - Lagerstätten ausgezeichnet, die für die unterirdische Speicherung von CO2 durchaus geeignet erscheinen.

Beispielsweise könnte sich zwischen Mesekenhagen und Lüssow ein Speicher erstrecken, wobei eine CO2 - Pipeline zwischen Lubmin und Mesekenhagen eine Option darstellen könnte.

Im Sinne des vorgeschlagenen neuartigen Verfahrensweges wäre ein Kraftwerk mit moderner Oxyfuel-Technologie, die wahrscheinlich eine Luftzerlegungsanlage und CO2-Gewinnungsanlage beinhaltet, lediglich mit einer Elektrolyse und einer Methanolreaktor- und Rektifiziereinheit zu ergänzen und im Nebenschluss über eine Pipeline mit dem unterirdischen CO2-Speicher zu verbinden, um Methanol aus Erneuerbaren Energien herzustellen.

Beispielhaft sind ebenfalls Stadtwerke geeignet, um eine Kohlendioxid-Verwertung mit dem Vorteil einer Netzentlastung in der Regelzone Mecklenburg-Vorpommern (Vattenfall Europe Transmission) zu verknüpfen. Als Kohlendioxid-Quelle kommen ebenfalls Biogasanlagen in Frage, bei denen die Herstellung von Biogas mit Erdgasqualität erfolgt, wozu verfahrenstechnisch eine Abtrennung des Kohlendioxids vorgenommen wird.

## Patentansprüche

1. Verfahren zur Herstellung von Methanol durch die Verwertung von Kohlendioxid aus Abgas fossil befeuerter Kraftwerke, Heizkraftwerke und Emittenten, ausgewählt aus Zementwerken der Zementindustrie, chemischen Anlagen, Stahlwerken, Anlagen der Industrie, Biogas-und/oder Biomasseanlagen, wobei Kohlendioxid aus dem Abgas für eine Methanolsynthese mit Wasserstoff vermischt und unter Verwendung von Katalysatoren in Methanol umgesetzt wird, **dadurch gekennzeichnet, dass** eine technologische Einheit für eine Kohlendioxid-Verwertung und Wasserstoff-Methanolspeicherung (CV WMS) vorgesehen ist und die technologische Einheit der CO2-Verwertungs- und Wasserstoff-Methanol-Speicherung (CV WMS) bestehend aus
- Elektrolyse von Wasser mit H2- und O2-Gewinnung,
- CO2 - Ad- oder Absorption mit CO2-Gewinnung und
- Methanolsynthese mit MeOH-Speicherung,
containerisiert am Standort einer Kohlendioxid-Quelle zur Verfügung gestellt wird.

2. Verfahren nach Anspruch 1, **dadurch gekennzeichnet, dass**
das Kohlendioxid aus dem Abgas der Methanolsynthese direkt zugeführt wird und/oder das Kohlendioxid aus Speichern der Methanolsynthese indirekt zugeführt wird.

3. Verfahren nach Anspruch 1 oder 2, **dadurch gekennzeichnet, dass**
das Kohlendioxid aus dem Abgas der fossil befeuerten Kraftwerke absorbiert oder adsorbiert wird.

4. Verfahren nach einem oder mehreren der vorhergehenden Ansprüche,
**dadurch gekennzeichnet, dass**
das hergestellte Methanol in den Feuerungsprozess von Kraftwerken zur Stromerzeugung zurückgeführt wird, um Windflauten auszugleichen und dass das dabei frei werdende Kohlendioxid erneut zur Synthese von Methanol eingesetzt wird.

5. Verfahren nach Anspruch 4, **dadurch gekennzeichnet, dass** eine Steuerung vorgesehen ist, mittels derer die Erzeugung der Elektroenergie bedarfsgerecht gesteuert wird, wobei bei nicht ausreichenden Aufkommen aus Wind- , Wasser- und/oder Solarenergieerzeugungsanlagen die fossilen Energieerzeugungsanlagen mit Methanol aus dem Methanol-Speicher für die Erzeugung von Elektroenergie unterstützt werden.

6. Energieerzeugungsanlage mit wenigstens einem mit fossilen Wärmeerzeugungsträgem befeuerbaren Heizkraftwerk, wenigstens einer Dispatcher- oder Verteilerstation, wenigstens einem mit Wind- und/oder Wasserkraft betriebenen Kraftwerk und/oder einem Solarkraftwerk, wenigstens einer Elektrolyseanlage zur Spaltung von Wasser in Wasserstoff und Sauerstoff, wenigstens je einer Speicherstation für Kohlendioxid, Sauerstoff und Wasserstoff sowie wenigstens einer Methanolerzeugungsanlage zur Durchführung einer Methanolsynthese, wenigstens einem Methanol-Speicher sowie einer Verknüpfung oder Vernetzung der einzelnen Anlagenbestandteile und einer Steuerung, die die Anlagenbestandteile zur Energieerzeugung in Abhängigkeit vom Strombedarf zur Optimierung der Auslastung zu steuern vermag, und die einzelnen Anlagenbestandteile an einem einzigen Standort vorgesehen sind, **gekennzeichnet dadurch, dass**
eine technologische Einheit einer CO2-Verwertung und Wasserstoff - Methanol - Speicherung (CV WMS) als eine containerisierte Einheit angeordnet ist, und diese aus
- einer Elektrolyse von Wasser mit H2 - und O2 -Gewinnung,
- einer CO2 - Ad- oder Absorption mit CO2 - Gewinnung und
- einer Methanolsynthese mit MeOH - Speicherung besteht,
die Anlagenteile der technologische Einheit der CO2-Verwertung und Wasserstoff-Methanol-Speicherung (CV WMS) in unterschiedlichen Modulen angeordnet sind, die durch Schnittstellen miteinander verbindbar sind, und
die Anlagenteile der technologische Einheit der CO2-Verwertung und Wasserstoff-Methanol-Speicherung (CV WMS) in einem Container angeordnet sind.

## Claims

1. Process for preparing methanol by utilization of carbon dioxide from exhaust gas from fossil fuel-fired power stations, combined heat and power stations, and emitters selected from among cement works of the cement industry, chemical plants, steel works, plants of industry, biogas and/or biomass plants, where carbon dioxide from the exhaust gas is mixed with hydrogen for a methanol synthesis and converted into methanol using catalysts, **characterized in that** a technological unit for carbon dioxide utilization and hydrogen-methanol storage (CV WMS) is provided and the technological unit for CO₂ utilization and hydrogen-methanol storage (CV WMS) consisting of
- electrolysis of water to give H₂ and O₂,
- CO₂ adsorption or absorption to recover CO₂ and
- synthesis of methanol with MeOH storage,
is provided containerized at the site of a carbon dioxide source.

2. Process according to Claim 1, **characterized in that** the carbon dioxide from the exhaust gas is fed directly to the methanol synthesis and/or the carbon dioxide is fed indirectly to the methanol synthesis from storages.

3. Process according to Claim 1 or 2, **characterized in that** the carbon dioxide from the exhaust gas from the fossil fuel-fired power stations is absorbed or adsorbed.

4. Process according to one or more of the preceding claims, **characterized in that** the methanol produced is recirculated to the firing process of power stations to generate electric power in order to equalize lulls in wind and **in that** the carbon dioxide liberated is reused for the synthesis of methanol.

5. Process according to Claim 4, **characterized in that** a control system by means of which the generation of electric energy is controlled according to requirements is provided, with in the case of insufficient supply from wind, water and/or solar energy generation plants the fossil energy generation plants being supplemented with methanol from the methanol storage for the generation of electric energy.

6. Energy generation plant having at least one combined heat and power station which is able to be fired with fossil heat carriers, at least one dispatcher or distributor station, at least one power station operated using wind power and/or water power and/or a solar power station, at least one electrolysis plant for splitting water into hydrogen and oxygen, at least one storage station for each of carbon dioxide, oxygen and hydrogen and also at least one methanol production plant for carrying out a methanol synthesis, at least one methanol storage and a connection or networking of the individual plant components and a control system which is able to control the plant components for energy generation as a function of the power demand in order to optimize the loading and the individual plant components are provided at a single site,
**characterized in that**
a technological unit for CO₂ utilization and hydrogen-methanol storage (CV WMS) is arranged as a containerized unit and this consists of
- an electrolysis of water to give H₂ and O₂,
- a CO₂ adsorption or absorption with recovery of CO₂ and
- a methanol synthesis with MeOH storage,
the plant parts of the technological unit for CO₂ utilization and hydrogen-methanol storage (CV WMS) are arranged in different modules which can be connected to one another via interfaces and
the plant parts of the technological unit for CO₂ utilization and hydrogen-methanol storage (CV WMS) are arranged in a container.

## Revendications

1. Procédé pour la préparation de méthanol par la valorisation de dioxyde de carbone provenant de gaz d'échappement de centrales à combustion de combustible fossile, de centrales thermiques et d'émetteurs, choisis parmi des cimenteries de l'industrie cimentière, des installations chimiques, des aciéries, des installations industrielles, des installations de biogaz et/ou de biomasse, le dioxyde de carbone du gaz d'échappement, pour une synthèse de méthanol, étant mélangé avec de l'hydrogène et étant transformé en méthanol par l'utilisation de catalyseurs, **caractérisé en ce qu'**une unité technologique pour une valorisation du dioxyde de carbone et le stockage de l'hydrogène et du méthanol (CV WMS) est prévue et l'unité technologique pour une valorisation du CO₂ et le stockage de l'hydrogène et du méthanol (CV WMS) étant constituée de
- électrolyse de l'eau avec obtention de H₂ et O₂,
- adsorption ou absorption de CO₂ avec obtention de CO₂ et
- synthèse de méthanol avec stockage de méthanol, est mise à disposition en conteneurs sur le lieu d'une source de dioxyde carbone.

2. Procédé selon la revendication 1, **caractérisé en ce que** le dioxyde de carbone provenant du gaz d'échappement est introduit directement dans la synthèse de méthanol et/ou le dioxyde de carbone de stocks est introduit indirectement dans la synthèse de méthanol.

3. Procédé selon la revendication 1 ou 2, **caractérisé en ce que** le dioxyde de carbone provenant de gaz d'échappement des centrales à combustion de combustible fossile est absorbé ou adsorbé.

4. Procédé selon l'une quelconque ou plusieurs des revendications précédentes, **caractérisé en ce que** le méthanol produit est recyclé dans le processus de combustion de centrales pour la production d'électricité, pour compenser des absences de vent et que le dioxyde de carbone libéré par-là est à nouveau utilisé pour la synthèse de méthanol.

5. Procédé selon la revendication 4, **caractérisé en ce qu'**un dispositif de régulation est prévu, à l'aide duquel la production de l'énergie électrique est réglée en fonction des besoins, où, lors de productions insuffisantes à partir d'installations de production d'énergie éolienne, hydroélectrique et/ou solaire, les installations de production d'énergie fossile sont soutenues par du méthanol provenant du stockage de méthanol, pour la production d'énergie électrique.

6. Installation de production d'énergie comportant au moins une centrale thermique qui est combustible avec dispositifs générant de la chaleur fossile, au moins une station de répartiteur ou une station de distributeur,au moins une centrale éolienne et/ou hydroélectrique et/ou une centrale solaire, au moins une centrale d'électrolyse pour la séparation de l'eau en hydrogène et en oxygène, au moins à chaque foisune station pour le stockage de dioxyde de carbone, d'oxygène et d'hydrogène ainsi qu'au moins une centrale de production de méthanol destinée à la réalisation d'une synthèse de méthanol, au moins un stockage de méthanol ainsi qu'un lien ou une mise en réseau des composants individuels de la centrale et un dispositif de régulation, qui est capable de réguler les composants de la centrale pour la production d'énergie en fonction de la demande en électricité afin d'optimiser le taux d'utilisation, et les composants individuels de la centrale étant prévus d'être localisés en un seul endroit, **caractérisée en ce que**
une unité technologique d'une valorisation du CO₂ et de stockage de l'hydrogène et du méthanol (CV WMS) est disposée en tant qu'unité en conteneurs,
et celle-ci est constituée de
- uneélectrolyse de l'eau avec obtention de H₂ et O₂,
- uneadsorption ou absorption de CO₂ avec obtention de CO₂ et
- unesynthèse de méthanol avec stockage de MeOH, les parties d'installation de l'unité technologique de la valorisation du CO₂ et de stockage de l'hydrogène et du méthanol (CV WMS) sont disposées en différents modules, qui peuvent être connectés entre eux par des interfaces, et les parties d'installation de l'unité technologique de la valorisation du CO₂ et de stockage de l'hydrogène et du méthanol (CV WMS) sont disposées dans un conteneur.
